(19) Europäisches Patentamt

European Patent Office

Office européen des brevets

(11) Veröffentlichungsnummer : **0 497 754 A1**

(12) # EUROPÄISCHE PATENTANMELDUNG

(21) Anmeldenummer : **92890020.8**

(22) Anmeldetag : **24.01.92**

(51) Int. Cl.$^5$ : **B08B 17/02**, C02F 1/48, A61N 5/00

(30) Priorität : **28.01.91 AT 185/91**

(43) Veröffentlichungstag der Anmeldung :
**05.08.92 Patentblatt 92/32**

(84) Benannte Vertragsstaaten :
**AT BE CH DE DK ES FR GB GR IT LI NL SE**

(71) Anmelder : **Grander, Johann
Haus Ingeborg Nr. 370
A-6373 Jochberg (AT)**

(72) Erfinder : **Grander, Johann
Haus Ingeborg Nr. 370
A-6373 Jochberg (AT)**

(74) Vertreter : **Puchberger, Peter, Dipl.-Ing. et al
Patentanwälte Dipl.-Ing. Georg Puchberger
Dipl.-Ing. Rolf Puchberger Dipl.-Ing. Peter
Puchberger Singerstrasse 13
A-1010 Wien (AT)**

(54) **Anordnung zur Verhinderung oder Entfernung von Ablagerungen in Rohren.**

(57) Anordnung zur Verhinderung oder Entfernung von Ablagerungen in Leitungsrohren oder Verunreinigungen in strömenden Medien wie Wasser, wässrigen Lösungen oder Gas, dadurch gekennzeichnet, daß eine von dem strömenden Medium (1,9) durchströmte Kammer (2,8) und mindestens eine mit einem als Anregungsmedium wirkenden ruhenden Medium (4) gefüllte Kammer (3,10,11) vorgesehen sind, wobei bei dem Anregungsmedium die elektromagnetische Struktur durch Änderung der magnetischen Kernresonanzeigeschaften und durch Ausbildung von supermolekularen Komplexen zwischen den Molekülen der Schwingungszustand geändert ist.

FIG.1

EP 0 497 754 A1

Die Erfindung betrifft eine Anordnung zur Verhinderung und Entfernung von Ablagerungen in Leitungsrohren oder Verunreinigungen in strömenden Medien wie Wasser, wässrige Lösungen oder Gas.

Es ist bekannt, Flüssigkeiten primär zu aktivieren, wobei durch Umwandlung eines Teiles der inneren Energie, unter Abkühlung und Eintrag von behanlungsenergie, ein höherer Energiegehalt aufgeprägt wird. So ist es auch gelungen, Wasser in seiner elektromagnetischen Struktur so umzuwandeln, daß sowohl durch Modifikation der magnetischen Kernresonanzeigenschaften (Änderung der Spin-Spin-Kopplungs-konstanten) als auch durch Induktion der Ausbildung von supermolekularen Komplexen zwischen einzelnen Wassermolekülen der elektromagnetische Schwingungszustand modifiziert wurde. Diese elektromagnetischen Schwingungen sind verantwortlich für das Entstehen eines elektromagnetischen Feldes von definierter Frequenz, Phase und Amplitude, welche sich in alle Richtungen gleichmäßig ausbreitet. Überraschenderweise hat es sich gezeigt, daß größere Partikel und Molekülgruppen in einem derartigen elektromagnetischen Feld in kleine Molekülgruppen zerlegt werden, wodurch unter anderem die Viskosität verringert wird, Schadstoffmoleküle aufgelöst werden können und dgl. mehr. Höchst überraschend ist auch, daß der energetische Zustand der aktivierten Flüssigkeiten beibehalten wird, d.h. nicht abklingt.

Bei Leitungen für strömende Medien wie z.B. Wasserleitungen besteht stets das Problem der Verhinderung von Ablagerungen von Salzen Kalk oder Rost. Handelt es sich bei den Leitungen um Gefäße von Lebewesen wie z.B. Blutbahnen oder Bahnen anderer Körpersäfte, dann kann es auch in diesen Leitungen zu schädlichen Ablagerungen kommen. Auch solche Ablagerungen sollen verhindert werden. Bei gasförmigen strömenden Medien wie z.B. Luft stellt sich die Aufgabe, Verunreinigungen zu entfernen oder diese so aufzuspalten, daß eine Reinigung leichter erzielbar ist.

Überraschenderweise hat sich gemäß vorliegender Erfindung gezeigt, daß durch die Einwirkung der zuvor beschriebenen aktivierten Flüssigkeiten auf derartige Rohr- oder Leitungssysteme die gewünschten Effekte erzielt werden können. Bei Wasserleitungen ist es so möglich, die Ablagerung von Kalk, anderen Salzen oder Rost zu verhindern, sowie vorhandene Ablagerungen aufzulösen. Bei Einwirkung auf gasförmige Medien wie Luft werden Verunreinigungen aufgespalten, sodaß sie enfernt oder Leichter entfernbar sind. Bei organischen strömenden Medien wie Blut oder andere Körpersäfte, in Adern und anderen Leitungsbahnen des Körpers werden ebenfalls Kalk- und Salzablagerungen verhindert und die Körpersäfte werden harmonisiert und normalisiert.

Die erfindungsgemäße Anordnung der eingangs gennanten Art ist dadurch gekennzeichnet, daß eine von dem strömenden Medium durchströmte Kammer und mindestens eine mit einem als Anregungsmedium wirkenden ruhenden Medium gefüllte Kammer vorgesehen sind, wobei bei dem Anregungsmedium die elektromagnetische Struktur durch Änderung der magnetischen Kernresonanzeigenschaften und durch Ausbildung von supermolekularen Komplexen zwischen den Molekülen der Schwingungszustand geändert ist.

Weitere vorteilhafte Merkmale sind den Unteransprüchen, der nachfolgenden Beschreibung und den Zeichnungen zu entnehmen.

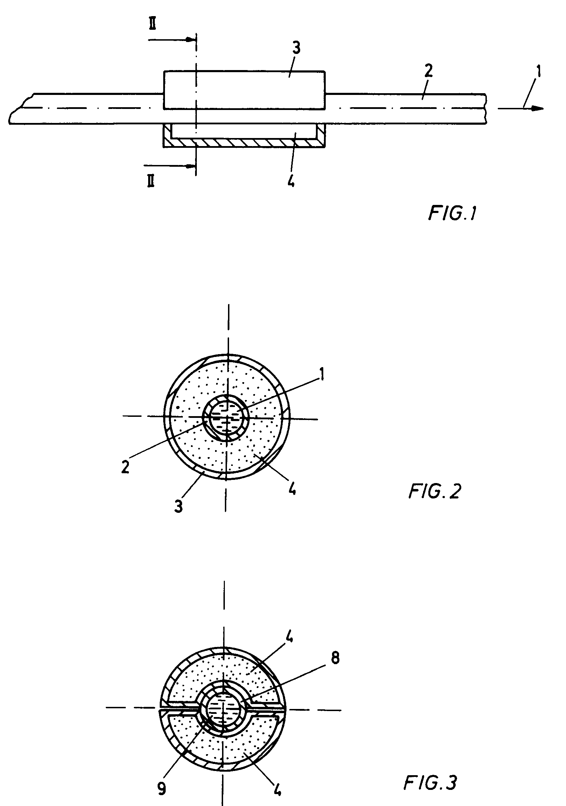

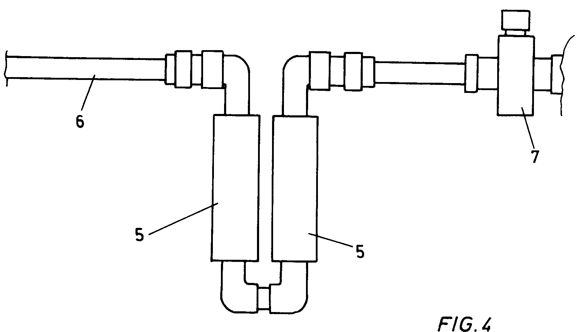

Fig.1 zeigt die erfindungsgemäße Anordnung in einer Ausführungsvariante teilweise geschnitten. Fig.2 ist ein Schnitt nach der Linie II-II in Fig.1 und Fig.3 ein ähnlicher Schnitt durch eine andere Ausführungsvariante. Fig.4 zeigt die Ansicht einer Ausführung der erfindugsgemäßen Anordnung in einer Wasserleitung.

Fig.1 zeigt als Kammer, die von dem strömenden Medium 1 durchströmt wird, ein Rohr 2, auf dem Mantelrohr 3 aufsitzt. Dieses Mantelrohr 3 bildet die Kammer für das Anregungsmedium 4.

Somit umgibt das Anregungsmedium das Leitungsrohr 2 über einen bestimmten Teil seiner Länge. Beim Durchströmen des strömenden Mediums 1 wirkt das Anregungsmedium 4 auf das strömende Medium ein, wodurch die vorteilhaften Effekte im strömenden Medium und im nachfolgenden Leitungssystem hervorgerufen werden.

In den Fig. 1 und 2 ist das Leitungsrohr durchgehend angeordnet, während das Mantelrohr 3 auf dem Leitungsrohr aufsitzt. In bevorzugter Weise kann die Anordnung als Baueinheit 5 vorgesehen werden, wobei die Baueinheit sowohl das Mantelrohr 3 als auch die Kammer für das durchströmende Medium und entsprechende Anschlußstücke für die Leitungsrohre aufweist. In der Ausführung Fig.4 sind zwei derartiger Baueinheiten 5 seriell mit Rohrkrümmern in eine Leitung 6 eingebaut. Mit 7 ist ein Druckreduzierventil bezeichnet, wie es in Wasserleitungen üblicherweise eingebaut wird. Die erfindungsgemäßen Baueinheiten 5 können in beleibiger Zahl seriell hintereinander oder parallel zueinander in eine Rohrleitung eingebaut werden.

Fig.3 zeigt ein weiteres Ausführungsbeispiel für die erfindungsgemäße Anordnung. Der Mantel mit dem Anregungsmedium 4 ist eine von der Kammer 8 für das strömende Medium 9 getrennte Baueinheit, wobei die Kammer des Anregungsmediums an die vom strömenden Medium durchströmte Kammer anlegbar ist. In der beispielsweisen Auführung Fig.3 wird das Rohr 8 von zwei halbschalenförmigen Mantelrohren 10,11 eingeschlossen.

Anstelle des zentralen Rohres 8 kann z.B. auch eine Körperteil vorgesehen sein, auf den eine oder mehrere Kammern mit Anregungsmedium aufgelegt werden. Bei dieser Anordnung werden die zuvor beschriebenen vorteilhaften Einwirkungen auf die Körpersäfte wie Blut oder Lymphflüssigkeiten erzielt. Die Kammer des Anregungsmediums kann auch aus flexiblen Material bestehen, um das anlegen an körperteile zu erleichtern.

Als Anregungsmedium wird bevorzugt aktiviertes Wasser eingesetzt, das in einem Gravitationsenergiewandler aktiviert wurde. Diese Gravitationsenergiewandler sind an und für sich bekannt und benutzen Gravitationsmagnetfelder. Bekanntlich baut ein Gravitationsenergiemagnetfeld um sich Teilchenströme auf, die sich mit einer ganz bestimmten Geschwindigkeit bewegen. Diese Teilchenströme, welche von feinstoffähnlicher Art sind, können die herkömmlichen Eisen- und Elektromagnetfelder nicht beeinflussen. Im Gravitationsenergiewandler wird nun durch speziellen Magnetmaterialinhalt und durch die spezielle Anordnung dieser Magnete in einer Scheibe eine Umlenkung der Teilchen erzwungen. Diese Umlenkung der Teilchen in eine neue Bahn läßt im Mittelpunkt einen erhöhten Teilchendruck entstehen und es wird "verdichtete Energie" gebildet.

## Patentansprüche

1. Anordnung zur Verhinderung oder Entfernung von Ablagerungen in Leitungsrohren oder Verunreinigungen in strömenden Medien wie Wasser, wässrigen Lösungen oder Gas, dadurch gekennzeichnet, daß eine von dem strömenden Medium (1,9) durchströmte Kammer (2,8) und mindestens eine mit einem als Anregungsmedium wirkenden ruhenden Medium (4) gefüllte Kammer (3,10,11) vorgesehen sind, wobei bei dem Anregunsmedium die elektromagnetische Struktur durch Änderung der magnetischen Kernresonanzeigeschafter und durch Ausbildung von supermolekularen Komplexen zwischen den Molekülen der Schwingungszustand geändert ist.

2. Anordnung nach Anspruch 1, dadurch gekennzeichnet, daß die Kammer (3,10,11) für das Anregungsmedium (4) die Kammer (2,8) für das strömende Medium (1,9) umgibt.

3. Anordnung nach Anspruch 1 oder 2, dadurch gekennzeichnet, daß das Anregungsmedium aktiviertes Wasser ist, das ein elektromagnetisches Feld definierter Frequenz, Phase und Amplitude aufweist.

4. Anordnung nach einem der Ansprüche 1 bis 3, dadurch gekennzeichnet, daß sie als Kammer (2,8) für das strömende Medium ein Rohr und als Kammer (3,10,11) für das Anregungsmedium ein das Rohr zumindest teilweise umgebendes Mantelrohr aufweist.

5. Anordnung nach Anspruch 4, dadurch gekennzeichnet, daß sie als Baueinheit (5) ausgebildet ist, die einzeln oder mehrfach seriell oder parallel in eine Rohrleitung einbaubar ist (Fig. 4).

6. Anordnung nach einem der Ansprüche 1 bis 3, dadurch gekennzeichnet, daß die das Anregungsmedium enthaltende Kammer (10,11) oder mehrere derartige Kammern an die vom strömenden Medium durchströmte Kammer (8) anlegbar ist (sind).

7. Anordnung nach einem der vorhergehenden Ansprüche, dadurch gekennzeichnet, daß das strömende Medium Wasser oder eine wässrige Lösung ist, und die Ablagerung oder Bildung von Salz-, Kalk- oder Rostabalgerungen verhindert oder verminder wird.

8. Anordnung nach einem der Ansprüche 1 bis 7, dadurch gekennzeichnet, daß als strömendes Medium Körpersäfte wie Blut oder Plasma und als Kammer (3,10,11) für diese strömenden Medien die körpereigenen Leitungsbahnen (2,8) vorgesehen sind.

9. Anordnung nach einem der Ansprüche 1 bis 8, dadurch gekennzeichnet, daß die Kammer des Anregunsmedium aus flexiblem Material besteht.

10. Anordnung nach einem der vorhergehenden Ansprüche 1 bis 6, dadurch gekennzeichnet, daß das strömende Medium Luft ist, um diese zu reiningen oder die Reinigung zu erleichtern.

FIG.1

FIG.2

FIG.3

FIG. 4

| Europäisches Patentamt | **EUROPÄISCHER RECHERCHENBERICHT** | Nummer der Anmeldung |
| --- | --- | --- |
| | | EP 92 89 0020 |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
| --- | --- | --- | --- |
| X<br>A | EP-A-0 389 888 (GRANDER) 3. Oktober 1990<br>* Seite 2, Zeile 5 - Zeile 20 *<br>* Seite 2, Zeile 25 - Zeile 36 *<br>--- | 1-5<br>6,7,8,10 | B08B17/02<br>C02F1/48<br>A61N5/00 |
| X<br><br>A | EP-A-0 198 958 (RHEINMAGNET HORST BAERMANN GMBH) 29. Oktober 1986<br>* Seite 5 - Seite 12; Abbildungen 1-12 *<br>--- | 1,2,4,5,<br>6,8,9<br>3,7 | |
| X<br><br>A | EP-A-0 319 936 (SCHUBERT) 14. Juni 1989<br><br>* Spalte 2, Zeile 28 - Spalte 3, Zeile 9; Abbildungen 1-5 *<br>--- | 1,2,4,5,<br>6<br>3,7 | |
| X<br><br>A | WO-A-8 102 529 (WHITE LIGHT INDUSTRIES INC.) 17. September 1981<br>* Seite 4 - Seite 9; Abbildungen 1-6 *<br>--- | 1,2,4,5,<br>6<br>3,7 | |
| X | US-A-2 829 636 (HENSCHKE) 8. April 1958<br><br>* Spalte 2, Zeile 55 - Spalte 6, Zeile 17; Abbildungen 1-8 *<br><br>----- | 1,2,4,5,<br>8,9 | RECHERCHIERTE SACHGEBIETE (Int. Cl.5)<br><br>B08B<br>C02F<br>A61N<br>F24D<br>F02B |

Der vorliegende Recherchenbericht wurde für alle Patentansprüche erstellt

| Recherchenort | Abschlußdatum der Recherche | Prüfer |
| --- | --- | --- |
| DEN HAAG | 18 MAI 1992 | VOLLERING J.P.G. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
 
& : Mitglied der gleichen Patentfamilie, übereinstimmendes Dokument

EPO FORM 1503 03.82 (P0403)